(19) **Europäisches Patentamt / European Patent Office / Office européen des brevets**

(11) **EP 3 098 597 B1**

(12) **EUROPEAN PATENT SPECIFICATION**

(45) Date of publication and mention
of the grant of the patent:
**15.09.2021 Bulletin 2021/37**

(21) Application number: **14844970.5**

(22) Date of filing: **23.12.2014**

(51) Int Cl.:
*G01N 27/02* (2006.01)     *G01N 33/12* (2006.01)

(86) International application number:
**PCT/ES2014/070972**

(87) International publication number:
**WO 2015/107234 (23.07.2015 Gazette 2015/29)**

(54) **METHOD FOR DETERMINING QUALITY PARAMETERS IN MEAT PRODUCTS**

VERFAHREN ZUR BESTIMMUNG VON QUALITÄTSPARAMETERN IN FLEISCHPRODUKTEN

PROCÉDÉ POUR DÉTERMINER DES PARAMÈTRES DE QUALITÉ DANS DES PRODUITS CARNÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.01.2014 ES 201430053**

(43) Date of publication of application:
**30.11.2016 Bulletin 2016/48**

(60) Divisional application:
**18184665.0 / 3 425 380**

(73) Proprietor: **Lenz Instruments S.L.
08940 Cornellà de Llobregat (ES)**

(72) Inventors:
- **JUAN MANUEL, Rodríguez Ventura
08018 Barcelona (ES)**
- **JACOBO, Álvarez García
08012 Barcelona (ES)**
- **MARK RICHARD, Williams Hallows
08173 Sant Cugat del Vallès (ES)**

(74) Representative: **Curell Suñol S.L.P.
Muntaner 240, 4o 2a
08021 Barcelona (ES)**

(56) References cited:
| | |
|---|---|
| EP-A2- 0 375 366 | WO-A2-2013/050986 |
| WO-A2-2013/050986 | FR-A1- 2 843 636 |
| GB-A- 1 436 900 | RU-C2- 2 440 571 |
| RU-C2- 2 440 571 | |

- LEE S ET AL: "Use of electrical conductivity to predict water-holding capacity in post-rigor pork", MEAT SCIENCE, ELSEVIER SCIENCE, GB, vol. 55, no. 4, 1 August 2000 (2000-08-01), pages 385-389, XP027363066, ISSN: 0309-1740 [retrieved on 2000-08-01]
- E. FULLADOSA ET AL: "Estimation of dry-cured ham composition using dielectric time domain reflectometry", MEAT SCIENCE, vol. 93, no. 4, 1 April 2013 (2013-04-01), pages 873-879, XP055059060, ISSN: 0309-1740, DOI: 10.1016/j.meatsci.2012.12.002
- LEE S ET AL: "Use of electrical conductivity to predict water-holding capacity in post-rigor pork", MEAT SCIENCE, ELSEVIER SCIENCE, GB, vol. 55, no. 4, 1 August 2000 (2000-08-01), pages 385-389, XP027363066, ISSN: 0309-1740 [retrieved on 2000-08-01]
- E. Fulladosa ET AL: "Estimation of dry-cured ham composition using dielectric time domain reflectometry", Meat Science, vol. 93, no. 4, 1 April 2013 (2013-04-01), pages 873-879, XP055059060, ISSN: 0309-1740, DOI: 10.1016/j.meatsci.2012.12.002

EP 3 098 597 B1

## Description

### Field of the invention

The invention relates to a method for determining quality parameters in meat products according to the preamble of claim 1.

### Definitions

The term "meat products" in the present invention must be interpreted in a broad sense. In this sense, the concept includes all fresh, processed or semi-processed products of the meat industry, including meat of pork, veal, lamb, chicken, turkey and horse. For example, in the case of pork, the invention can be applied to cuts of meat that result from cutting (such as hams, shoulders, bellies, tenderloins or others), meat trimmings, minced meat or any other specialty in the charcuterie sector. In addition, the term "meat product" must be considered as the meat from any animal intended for human consumption, such as fish meat.

In the invention, the term "salt uptake in lean" relates to the amount of salt taken up in the lean of the meat product after being subjected to a dry salting process (as in the case of the Spanish cured ham) or in brine (such as applying a brine injection for cooked ham, for example).

Furthermore, in the invention the term "intramuscular fat" relates to the fat that has infiltrated lean tissue muscle fibers.

Additionally, in order to avoid any ambiguity, the following definitions are provided:

- Primary electromagnetic field: electromagnetic field generated in space by the at least one transmitter coil as a result of the circulation of a current having a time-variable amplitude in said transmitter coil.
- Secondary electromagnetic field: electromagnetic field generated in space by the currents induced in the meat product as a result of being subjected to the action of the primary electromagnetic field.
- Total electromagnetic field: electromagnetic field that results from the superposition of the primary electromagnetic field and secondary electromagnetic field and is detected based on the current induced in said at least one receiver coil.
- In-phase and quadrature components of the total electromagnetic field: components resulting from the vector decomposition of the total electromagnetic field, and having a 0° and 90° phase shift, respectively, relative to the primary electromagnetic field.

### State of the art

In addition to the chemical composition of a meat product in terms of its total fat and lean content, there are a number of variables relating to its final quality. Among such variables, the following group of parameters related in a complex manner with both the physicochemical characteristics of the meat product and with its tissue and cell structure, must be pointed out: (a) water-holding capacity, also referred to as WHC, (b) salt uptake in lean, defined based on the amount of salt taken up in the product muscle tissue (lean) upon subjecting it to a salting process and (c) the intramuscular fat content in the product.

In general, the relationship between these quality parameters and physicochemical and structural properties of the product is complex. In the case of water-holding capacity, this parameter is fundamentally conditioned by a series of processes, such as chemical decomposition of glycogen present in the muscle after slaughter, the formation of lactic acid, and evolution of muscle tissue pH. Associated with said changes, changes take place in the cell structure of the meat product on a cell membrane level. Such physicochemical processes and structural modifications ultimately determine the capacity of meat product to hold water within its structure.

Similarly, a meat product salting process also entails significant variations in its properties. At the physicochemical level, this process progresses through a dual diffusion process, whereby salt diffuses into the product and water diffuses towards the surface. Furthermore, as a result of the interaction of muscle tissue with salt ions and additives that are introduced, chemical and enzymatic reactions, as well as changes in the cell structure of the product, take place.

Finally, the intramuscular fat content of a meat product is directly related to the distribution of fat and muscle tissue in the product. Many times, as in the case of Iberian cured hams, the distribution of intramuscular fat is not homogenous and does not have a direct relationship with the total fat content of the product.

In the meat industry, determining the quality parameters of meat products (water-holding capacity, salt uptake in lean and intramuscular fat content) is extremely important for being able to adjust production processes and assure uniform product quality. Furthermore, this allows establishing production segmentation strategies, and in many cases, maximizing industrial output of the production process. Currently, these quality parameters can only be monitored by means of destructive and/or invasive methods. These methods have a number of limitations in terms of analysis time, automation for being implemented on the line, cost, maintenance, risk of cross contamination, and/or precision. Despite such limitations, the complex relationship between said quality parameters and product properties has hindered development of non-destructive inspection methods.

Among the quality parameters that are considered, water-holding capacity is particularly relevant because it has a considerable effect on both the organoleptic quality of the end product, and on the output of many industrial production processes. Raw material having insufficient water-holding capacity, such as pale, soft and exudative meats (PSE), or having excessive water-holding capacity, such as dark, firm and dry meats (DFD), cause defects in either fresh or prepared end products. These defects lower organoleptic quality of the product and reduce industrial output. The main defects are excessive weight loss, texture problems, food stability and safety problems, discolouration, inadequate salt uptake, defects when slicing the product, such as tears, cracks, holes, etc.

The water-holding capacity is estimated today by means of monitoring the progression of meat pH after slaughter, usually after 45 minutes ($pH_{45}$) and/or 24 hours ($pH_{24}$). Monitoring is done by means of needle shaped electrodes which are subjected to wear and eventual oxidation. Furthermore, this system has the added problem that the measurement is only representative of a limited volume of the meat product being analyzed.

Alternatively, in some cases the water-holding capacity can be estimated by means of measuring color in the lean area. The color can be objectively measured with a Minolta CR-400 colorimeter (or similar) or by means of a subjective visual inspection with well-trained operators. A limitation of the method of measuring by means of the colorimeter is the error introduced in the color measurement due to the presence of connective tissue and/or fat. On the other hand, this method alone does not allow determining a certain type of meats having a water-holding capacity problem, such as red, soft and exudative meats (RSE). This type of meat is present in a high percentage, around 35% of pork production.

In addition, and in relation to cured and salted product production processes, it is important to determine the amount of salt taken up by the processed meat for the purpose of assuring specific food quality and safety. Although there are a number of experimental methods for determining the content and distribution of salt in meat products, such as nuclear magnetic resonance imaging, most of them are complex and expensive to implement as quality control procedures in production lines. The instrument commonly used today in the meat industry is the salinometer, consisting of an invasive probe that allows evaluating conductivity of the cut. However, like in the case of measuring water-holding capacity, these measurements only provide information corresponding to a limited sample volume and are furthermore subject to errors associated with variability of the contact resistance between the electrode and the medium and with the geometry of the cut.

Another problem associated with measuring these quality parameters with the described probes consists of the difficulty in automating processes for determining these parameters.

In addition, the intramuscular fat content is an important quality parameter because it is closely related to the flavor, aroma and tenderness of the meat. A minimum amount of intramuscular fat (usually 2%) is necessary for obtaining optimal sensory quality of fresh meat. Usually, values comprised between 3 and 4% are more suitable for meat intended for cured products. In addition, in the production of cured meat (ham, tenderloin, etc.), intramuscular fat acts like a physical barrier to the release of water, thus preventing the cuts of meat from becoming excessively dehydrated. In addition, it also acts like a physical barrier against the entry of salt into the inner portions of the ham, which ultimately increases salting and curing times. Determining the intramuscular fat content in cuts of meat, in addition to enabling segmentation of the raw material and more optimally modifying production processes, allows meat processers to establish a fair payment criterion with the supplier of said raw material based on its quality.

Experimental methods for determining intramuscular fat are destructive, including chemical extraction or determination by means of near-infrared spectroscopy, also referred to as NIRS, among others.

Document EP 0375366 A2 discloses a method and an apparatus using a varying electromagnetic field for determining the nature, or a property of a nonmetallic, conductive material. In the apparatus a food sample is passed through a time-varying electromagnetic field generated by a transmitter coil whereby a signal is induced in a receiving signal due to the generation of eddy-currents. A balance coil offsets the large voltage induced in the receiving coil by the field alone and thereby improves the sensitivity of detection. A food sample has been found to generate both reactive and resistive components which can be detected by respective phase detectors that supply digital values to microprocessor and the reactive and resistive components can be used to provide a measure of freshness or ripening of e.g. vegetable matter. Adjustments for variations in the cross-sectional area of the sample are made with regard to a factor Kr which relates to the reactive and resistive components.

Summary of the invention

It is an object of the invention to provide a method of the type indicated above, to determine quality parameters in meat products by noninvasive methods and furthermore, to offer information that is more representative of the product as a whole. Particularly, the main objective of the invention is to determine the water-holding capacity and/or salt uptake in lean and/or intramuscular fat content, in any type of meat products, whether they are fresh or semi-processed cuts or cured or salted products.

This purpose is achieved by means of a method for determining quality parameters in meat products of the type indicated above, characterized by the features of claim 1.

Equivalently to determining the in-phase and quadrature components, the method could include determining the modulus and phase of the signal induced in the receiver coil or, where appropriate, multiple receiver coils.

The method according to the invention has multiple advantages with respect to the invasive methods. First, the meat products remain intact, and furthermore the risk of cross contaminations between different products is eliminated because it does not require probes to be inserted into the products. In addition, the mentioned problem of the overall estimate based on a local measurement is also solved because the present method allows analyzing the entire product as a whole. Furthermore, since the meat product is not handled with the probe, the system allows gaining inspection speed, thereby allowing implementation in completely automated sorting lines.

The described method is a powerful method of characterization, which allows determining quality parameters in meat products, as experimentally confirmed. In general, the relationship between said quality parameters and the electromagnetic response of the meat product is complex. First, it must be taken into account that it is not possible to establish a simple relationship between the quality parameters of the meat product and its physicochemical and structural properties. On the other hand, variations in the electromagnetic response of the product as a result of physicochemical and structural changes cannot be established in advance in a trivial manner either.

The method described in the present invention is based on analyzing variables affecting quality parameters in meat products, and on observing that these variables modify to a greater or lesser extent the magnitude and spatial distribution of the flow of currents induced in the meat product subjected to the action of a primary time-variable electromagnetic field. The developed technique preferably comprises using various excitation frequencies, which allows characterizing the response of the meat product giving priority to a specific mechanism of conduction. In general, working frequencies in the range of kHz (kilohertz) or tens of kHz are suitable for characterizing mechanisms of ion conduction, as well as the distribution and morphology of the product tissues. Complementarily, the frequency range of 100 kHz to 100 MHz (megahertz) allows characterizing the cell structure of the meat product through the mechanism of dispersion associated with the cell membrane of the constituent tissues, commonly known as mechanism of $\beta$-dispersion. Therefore, the use of multiple frequencies allows indirectly evaluating the physicochemical and structural properties of the meat product based on its electromagnetic response. The developed method is therefore based on experimentally establishing correlation models between the electromagnetic response of the meat product, through the in-phase and quadrature components of the total electromagnetic field, and the previously described quality parameters. It should also be pointed out that decomposition of the total electromagnetic field into its in-phase and quadrature components relative to the primary electromagnetic field is particularly advantageous, because it allows separating the contributions to the secondary electromagnetic field associated with the real part (in-phase component) and the imaginary part (quadrature component) of the dielectric constant of the meat product.

The invention further includes a number of preferred features that are object of the dependent claims and the utility of which will be highlighted hereinafter in the detailed description of an embodiment of the invention.

In a preferred embodiment, the method further comprises the steps of obtaining the initial weight of said meat product, adding said initial weight to said models of correlation and obtaining an estimated value of the water-holding capacity and/or salt uptake in lean and/or intramuscular fat content of said meat product based on said models of correlation. The initial weight can be obtained in a simple manner because it is either known at the entry into the installation or the installation can be equipped with a scale or weighing system. As an advantage to highlight, the initial weight parameter allows considerably improving the in-line estimate of the WHC, salt uptake in lean and intramuscular fat content. Such improvement is fundamentally related to the fact that mutual inductance between the meat product and the transmitter and receiver coils increases with the sample volume. In addition to this factor, it must also be taken into account that the amplitude of the secondary electromagnetic field increases with product mass. Therefore, the introduction of linear and/or non-linear terms including the weight of the product allows establishing more precise models of correlation.

In a preferred embodiment, the method further comprises the steps of obtaining weight loss after salting as a difference between the initial weight of said meat product and the final weight after salting, adding said weight loss to said models of correlation and obtaining an estimated value of the salt uptake in lean of said meat product based on said models of correlation. Weight loss after the salting process provides a direct measurement of the amount of water that the product has lost as a consequence of the diffusion of water towards the surface. Including this variable in models of correlation allows solving the uncertainty associated with the fact that the dielectric properties of the meat product depend on both salt uptake and on the drip loss in the diffusion process. Accordingly, the prediction error of the models for predicting salt uptake in lean considerably decreases.

In another preferred embodiment the method comprises the steps of obtaining the initial percentage by weight of lean and/or fat of said meat product adding said initial percentage by weight of lean and/or fat to said models of correlation and obtaining an estimated value of the water-holding capacity and/or salt uptake in lean and/or intramuscular fat content of said meat product based on said models of correlation. The importance of this additional parameter is based on the fact that the dielectric properties of the meat product, particularly at frequencies above 100 kHz, depend on the amount of muscle tissue present in the meat product. Therefore, normalizing the quadrature components to the lean tissue mass allows decoupling this effect, improving meat product quality parameter prediction capacity.

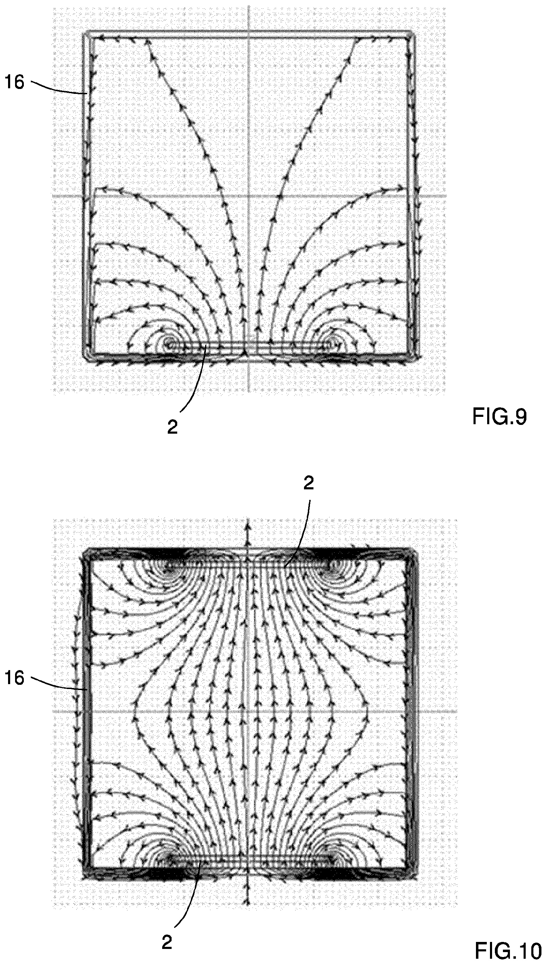

In addition, for the purpose of achieving greater directivity and homogeneity of the primary electromagnetic field, as well as greater directivity in detecting the total electromagnetic field, in a preferred embodiment the method comprises the step of providing first and second transmitter coils and first and second receiver coils, and the primary electromagnetic fields generated by said two transmitter coils have a 0 or 180° phase shift, depending on the winding direction of the wires thereof, and the estimate of the secondary electromagnetic field is made based on adding or subtracting the secondary electromagnetic fields obtained from said two receiver coils. In this configuration, the two transmitter coils are excited with signals having a 180° phase shift, such that the primary field consisting of the superposition of the fields of each of the transmitter coils has the particularity of being strongly confined in the space between the two coils. Similarly, improvement in directivity of the receiving system is achieved by subtracting the signals received in the two receiver coils. To graphically illustrate the effect of confining the primary electromagnetic field, Figure 10 shows a simulation of the distributions of the field lines obtained using a single transmitter coil, and two transmitter coils that have a 180° phase shift.

The method according to the invention comprises the steps of generating in said at least one transmitter coil a plurality of primary electromagnetic fields at different excitation frequencies, and determining the in-phase and/or quadrature components of the total electromagnetic fields for each of said excitation frequencies by means of said at least one receiver coil, obtaining a model of correlation comprising said in-phase and/or quadrature components of each of said total electromagnetic fields for each of said excitation frequencies, and obtaining an estimated value of the water-holding capacity and/or salt uptake in lean and/or intramuscular fat content of said meat product based on each of said models of correlation. In a particularly preferred manner, the method comprises implementing said at least one transmitter coil and said at least one receiver coil in the form of tunable coils, which allows obtaining the response of the meat product at multiple excitation frequencies, even in the case of using a single transmitter coil and a single receiver coil. It has additionally been found that excitation frequencies being comprised in a range comprised between 1 kHz to 100 MHz is particularly advantageous for the meat products.

Also for the purpose of calibrating the measurement of the phase and quadrature components, the method according to the invention comprises the steps of obtaining the total electromagnetic field in the absence of a meat product by means of determining in said at least one receiver coil the in-phase and quadrature components of the total electromagnetic field in the absence of said meat product, determining the phase shift between the total electromagnetic field and the primary electromagnetic field in the absence of said meat product, and correcting said phase shift.

Preferably and for the purpose of maximizing the dynamic measurement range of the instrument, the method also additionally comprises the steps of obtaining the total electromagnetic field in the absence of a meat product by means of determining in said at least one receiver coil the in-phase and/or quadrature component of said total electromagnetic field in the absence of said meat product, generating an electric compensation signal with a phase and a quadrature such that they cancel out the in-phase and quadrature components of the total electromagnetic field in said at least one receiver coil due to the primary electromagnetic field in the absence of a meat product. Comparatively, the primary electromagnetic field is much more intense than the secondary electromagnetic field generated in the meat product (usually, several orders of magnitude greater). Accordingly, in the absence of an electric compensation signal, the total electromagnetic field consists of the superposition of a component associated with the secondary electromagnetic field generated in the meat product and an additional component associated with the primary electromagnetic field, which conceals the contribution of interest associated with the secondary field. Therefore, as a result of the electric compensation signal, the signal detected in the receiver coil only corresponds to the signal produced by the meat product, and such signal can be freely amplified, taking advantage of the entire dynamic range of the acquisition system and substantially improving measurement precision.

In an alternative embodiment, the method additionally comprises the steps of obtaining the amplitude of the electric compensation signal needed for cancelling out the total electromagnetic field in the absence of a meat product, normalizing the values of the in-phase and quadrature components of the total field in the presence of a meat product by means of the process of dividing said in-phase and quadrature components by the value of said amplitude of the electric compensation signal, obtaining a model of correlation comprising said normalized in-phase and/or quadrature components of the total electromagnetic field for each of said excitation frequencies, and obtaining an estimated value of the water-holding capacity and/or salt uptake in lean and/or intramuscular fat content of said meat product based on each of said models of correlation. This method of normalization allows compensating for possible system drifts, improving measurement reproducibility and reliability.

Optionally, the method also comprises the steps of providing vision means, determining by means of said vision means at least one morphological parameter of said meat product selected from height, length, width, area and/or volume of said meat product, and adding one or several of said morphological parameters to said model of correlation, and obtaining an estimated value of the water-holding capacity and/or salt uptake in lean and/or intramuscular fat content of said meat product based on said models of correlation. Alternatively or complementarily to the initial weight of the meat product, the use of said morphological parameters allows improving the precision of correlation models, considering the relationship between the geometry of the meat product and the mutual induction coefficient between transmitter coil, meat product and receiver coil. Given a specific configuration, it is possible to establish terms for correcting models of correlation based on system response modeling either by means of analytical techniques and/or empirical methods based on finite element simulation.

Optionally, the method also comprises the steps of providing vision means, determining by means of said vision means at least one color parameter, expressed in the CIELAB color space, in the lean area of said meat product selected from luminance (L*) and/or the red/green level (a*) and/or the blue/yellow level (b*) of said meat product, and adding one or several of said color parameters (L*a*b*) to said model of correlation and obtaining an estimated value of the water-holding capacity of said meat product based on said models of correlation. The inclusion of said color coordinates improves the obtained correlation because during development of the invention, it has been found that this group of variables, particularly luminance (L*), is a good water-holding capacity estimator. The CIELAB color space refers to the color model used to describe all colors that can be perceived by the human eye.

In a particularly preferred manner, the method according to the invention comprises a prior image processing step consisting of removing from the image obtained by said vision means pixels corresponding to connective tissue and intramuscular fat before determining said color parameter.

Likewise, the invention also includes other features of detail illustrated in the detailed description of an embodiment of the invention and in the accompanying figures.

Brief description of the drawings

Further advantages and features of the invention will become apparent from the following description, in which, without any limiting character, preferred embodiments of the invention are disclosed, with reference to the accompanying drawings in which:

Figure 1 shows a longitudinally cut schematic side view of an installation for determining quality parameters in meat products.
Figure 2 shows a schematic front view of the installation of Figure 1.
Figure 3 shows a schematic front view of a second embodiment of the installation.
Figure 4 shows a longitudinally cut schematic side view of a third example of the installation.
Figure 5 shows a longitudinally cut schematic side view of a fourth example of the installation.
Figure 6 shows the estimated salt concentration values in lean in hams, obtained by means of a partial least squares regression (PLSR) model using 5 main components.
Figure 7 shows the estimated values of drip loss in tenderloins based on a PLSR model using 7 main components.
Figure 8 shows the estimated values of intramuscular fat in hams based on a PSLR model using 5 main components.
Figure 9 shows a simulation of the distribution of the primary electromagnetic field lines obtained from a single transmitter coil.
Figure 10 shows a simulation of the distribution of the primary electromagnetic field lines obtained from two transmitter coils having a 180° phase shift according to the arrangement of coils in Figure 5.

Detailed description of embodiments of the invention

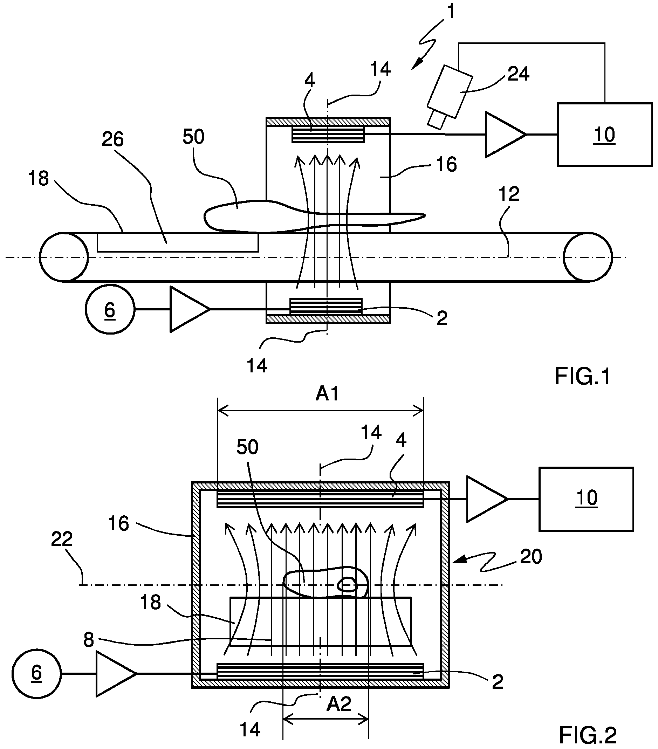

Figure 1 schematically shows an installation 1 for determining quality parameters in meat products.

The installation 1 has a longitudinal conveying direction 12 defined based on a conveyor belt 18 on which the meat product 50 rests, said meat product 50 in this embodiment being a ham. At the beginning of the conveyor belt 18, the installation comprises a scale 26 for determining the weight of the meat product 50 passing through the installation 1. Nevertheless, determining the weight in the production line is not essential for the invention because it could be done in another separate installation. It should also be mentioned that the conveyor belt 18 is not essential either because for small-capacity installations, such as installations intended for a laboratory, for example, the method according to the invention could be carried out in an interrupted manner in an installation not conveying the meat product.

In the vertical direction, a transmitter coil 2 is provided below the conveyor belt 18, whereas a receiver coil 4 is provided above it. In this embodiment, both coils 2, 4 are facing one another in the vertical direction of the installation. In addition, it can be seen that the winding axis 14 of the transmitter and receiver coils 2, 4 is transverse to the longitudinal direction 12, and in this case more specifically perpendicular to the plane going through the support surface for supporting the meat product 50 on the conveyor belt 18. The position of the transmitter and receiver coils 2, 4 could alternatively be interchanged, the receiver coil being provided above the conveyor belt 18 and the receiver coil 4 being provided below it.

As schematically indicated in Figures 1 and 2 through the field lines 8, this configuration generates a very homogenous electromagnetic field in the vertical direction, particularly in the central area of the coils 2, 4. Measurement errors due to variation of the cross section of the meat product 50 are therefore considerably reduced.

Figure 2 shows that the installation 1 also has generating means 6 connected to the transmitter coil 2. These generating means 6 will preferably be a multifrequency electric signal generator in order to carry out a frequency sweep, thereby improving characterization of the dielectric properties of the meat product 50 and obtaining an improvement in the prediction capacity of the models of correlation. The coils 2, 4 are also preferably tunable.

On the side of the receiver coil 4, the installation has determination means 10 for determining the in-phase and/or quadrature components of the total electromagnetic field relative to the primary electromagnetic field from the transmitter coil 2.

Finally, Figure 2 shows that the installation 1 comprises a magnetic circuit 16 having a square cross section, with the axis thereof being oriented parallel to the longitudinal direction 12, describing an O shape. The magnetic circuit 16 is of a material selected from a ferromagnetic, ferrimagnetic and superparamagnetic material, or combinations thereof.

As a result of its O-shaped configuration and the permeability characteristics of any of these three materials, the electromagnetic flux between the lower transmitter coil 2 and upper receiver coil 4 is confined in a closed inspection volume 20. The inspection volume 20 is as narrow as possible in the longitudinal direction 12. By way of example, Figure 9 schematically shows the effect of the magnetic circuit 16 with a transmitter coil 2. Said figure clearly shows that the primary electromagnetic field is confined in the inspection volume as a result of the action of a high-permeability ferrimagnetic shield ($\mu$=2000). Accordingly, as a result of the magnetic circuit 16 homogeneity of the electromagnetic field in the event of variations in dimension and/or position of the meat product 50 relative to the transverse axis 22 is improved, and the field is better directed. All this increases sensitivity by at least one order of magnitude and allows inspecting larger and more irregular cuts.

Also in Figure 2, the transmitter and receiver coils 2, 4 are rectangular and wider than the meat product 50, and in a particularly preferred manner at least 20% wider than the width of the meat product 50. They are furthermore arranged such that the width thereof is perpendicular to the longitudinal direction 12.

Finally, Figures 1 to 2 show that the installation 1 comprises vision means 24 in the form of cameras connected to the determination means 10. These cameras determine morphological parameters in the meat product 50, such as height, length, width, area and/or volume. As proposed by the method according to the invention, they can be added to the models of correlation for gaining precision in estimating quality parameters. The vision means 24 can be used in a complementary manner for determining color parameters of the meat product 50. In a particularly preferred manner, the color parameter is expressed in the CIELAB color space. This color parameter, in the lean area of the meat product 50 is selected from luminance L* and/or red/green level a* and/or blue/yellow level b* of the meat product 50. These color parameters L*, a*, b* can then be added to the model of correlation. An estimated value of the water-holding capacity of the meat product 50 can be obtained from this new, more precise model.

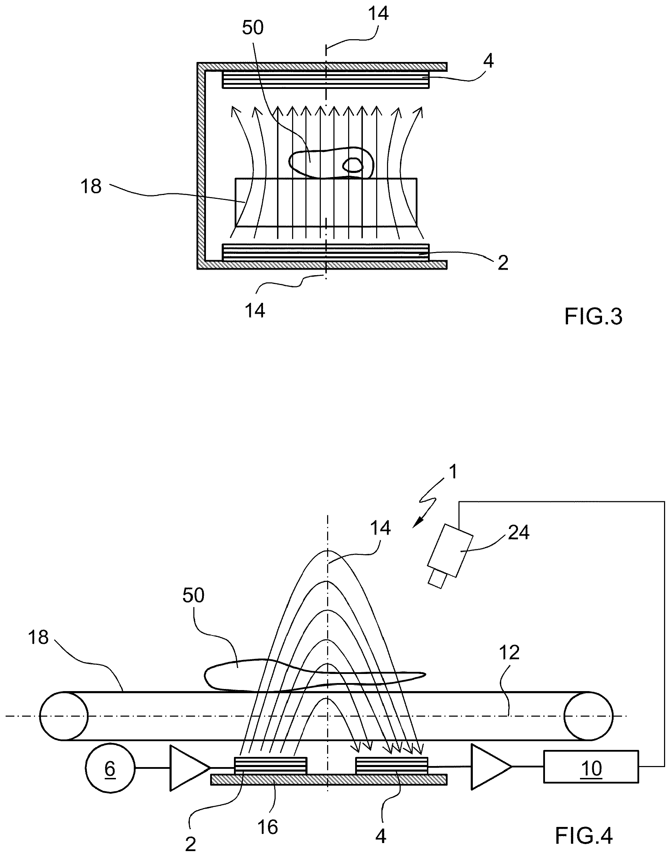

Figure 3 shows an alternative embodiment in which the magnetic circuit 16 is C-shaped, with the transmitter and receiver coils 2, 4 facing one another on both sides of the conveyor belt 18 in the vertical direction. The C-shaped configuration can obviously be both a conventional C shape and a Ɔ shape (left-inverted C shape), without this changing the scope of protection of the invention.

Figure 4 shows a third embodiment of the invention which is substantially similar to the embodiment in Figures 1 and 2. Nevertheless, in this case, the transmitter and receiver coils 2, 4 are both located below the conveyor belt 18 and connected to one another by means of the magnetic circuit 16. This offers flexibility to the installation 1 because as a result of this configuration, meat products having a larger volume can be analyzed without the limitation of them going through the magnetic circuit 16.

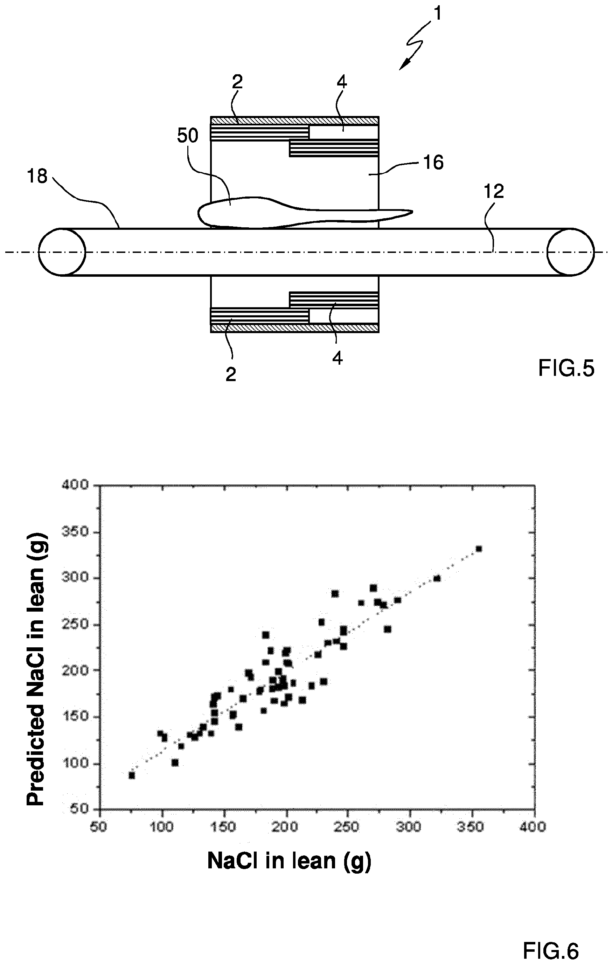

Figure 5 shows a fourth embodiment of the installation 1 in which two transmitter coils 2 and two receiver coils 4 are provided. One transmitter coil 2 and one receiver coil 4 are located in the lower part of the conveyor belt 18, whereas the other two coils 2, 4 are located in the upper part. Furthermore, each receiver coil 4 can be partially overlapped with its corresponding transmitter coil 2, which allows partially compensating for the field induced in the receiver coils 4 as a result of the primary field. This configuration allows cancelling the radial components of the electromagnetic field when the electric signals exciting the transmitter coils have a 180° phase shift, obtaining a very homogenous electromagnetic field in the axial direction of the coils, and confining it in the longitudinal direction. This effect can be more clearly seen in Figure 10, where the greater homogeneity of the electromagnetic field can be seen.

Figure 6 shows the results obtained with the method according to the invention in a first embodiment for determining the salt, NaCl, uptake in lean. The salt uptake in lean in sixty Parma hams was analyzed in this test.

The hams were subjected to an individual salting process for a period of 15 days. After salting with common salt, the hams were brushed and washed to remove salt residue on the surface. The salt, NaCl, uptake in lean was measured by means of analytical determination following standard protocols.

The initial weight W of the ham before salting was determined in a prior phase. Then weight loss WL after salting was determined as a difference between the initial weight W of the ham and the final weight after salting.

Next the hams were subjected in the installation 1 to the effects of the primary electromagnetic field generated by the transmitter coil 2. The electromagnetic response was obtained in the receiver coil 4 by determining the in-phase components I and quadrature components Q of the total electromagnetic field at two different frequencies: 1900 kHz and 725 kHz. Morphological parameters of the cuts (height, width, and maximum length) were also obtained by means of a viewing system.

The obtained results show that the in-phase and quadrature components of the total electromagnetic field can be used for establishing models of correlation for predicting salt uptake in lean. The inclusion of additional parameters also allows reducing the prediction error of models of correlation.

Remarkably, it has been found through experiments that it is possible to obtain a good correlation even by using only the quadrature component of the total electromagnetic field at 1.9 MHz and weight loss after salting. By way of illustration, the prediction equation obtained in this case based on a multilinear equation is provided below.

$$NaCl[g]=-35.5+31.17 \cdot WL[Kg]+2.06 \cdot Q[V]/W[kg]$$

Figure 6 shows the obtained curve from which salt uptake in lean can be determined using the preceding equation.

The use of advanced statistical methods such as partial least squares regression (PLSR) allows establishing more precise models of correlation, including the number of variables considered. Based on the analysis of the experimental results, it has been established that a model of correlation with 5 main components allows reducing the prediction error of salt uptake in lean to limits that are comparable to those of the reference method, with corrected coefficients of determination of Adj. $R^2$ = 0.86 and with a RMSE prediction error = 20 g.

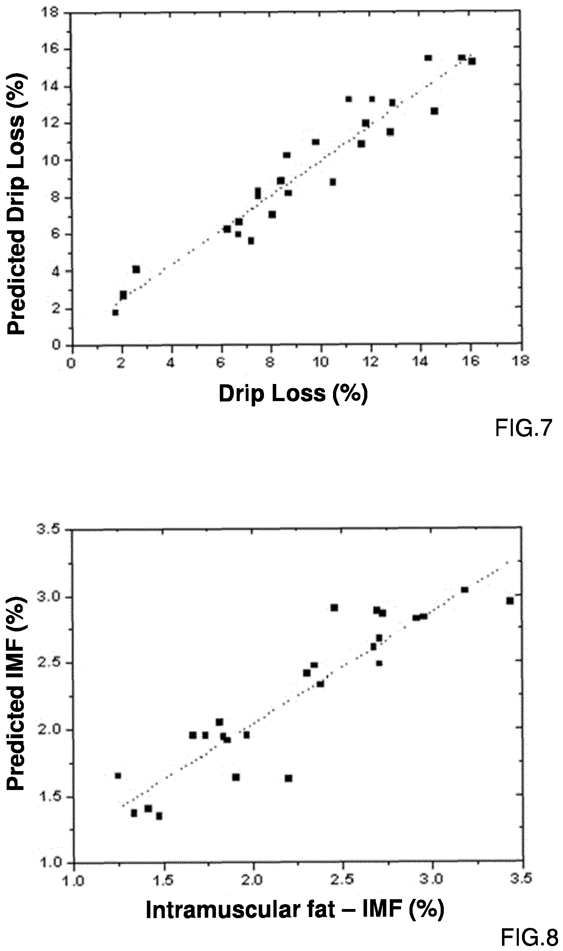

Figure 7 shows a second embodiment of the method according to the invention in the case of determining water-holding capacity.

In this second example, a total of 26 white pork tenderloin samples were analyzed. Water-holding capacity was determined by evaluating drip loss in 24 hours, following the normalized Honikel's Reference Bag Drip Method, considered to be the standard method in the industry for determining water-holding capacity.

The following parameters are determined in each case:

- Drip loss: DL%
- Weight of the cut: W
- CIELAB color coordinates of the lean surface (L*a*b*)
- Electric phase and quadrature signals at 10 different frequencies: { Ij Qj}

The excitation frequencies used were: 25 kHz, 40 kHz, 50 kHz, 85 kHz, 100 kHz, 200 kHz, 300 kHz, 400 kHz, 600 kHz and 850 kHz.

By means of models of linear correlation it can be seen that the induction spectrum is correlated to the target variable (DL%), and particularly the relationships between quadrature signals at different frequencies. For example, a simple linear model using only the weight and quotient Q100/Q40 provides correlations of Adj. $R^2$ = 0.76 and RMSE=2, Adj. $R^2$ being the adjusted correlation coefficient and RMSE being the root mean square error.

A multilinear model including the quotients Q100/40, Q300/85 and Q600/400 was used to improve the estimate of the water-holding capacity. Said model provides correlations of Adj. $R^2$ = 0.82 and RMSE=1.7.

The partial least squares (PLS) regression model, which includes all the experimentally determined variables ((Qi/Qj) [26x45], L*, a*, b*, w), is used to obtain an optimal model of correlation. It is established that the optimal number of variables in the model is 7 (number of main components).

The correlation achieved with the PLS model is Adj. $R^2$ = 0.94, with an RMSE calibration error =1%.

Figure 8 shows a third embodiment of the method according to the invention in the case of determining intramuscular fat.

In this third example, a total of 30 hams with a percentage of intramuscular fat between 1 and 10% were analyzed. The percentage of intramuscular fat was determined by means of the Soxhlet chemical extraction method, considered to be the standard method in the industry for determining fat content.

The following parameters were determined in each case:

- % of intramuscular fat (IMF): IMF%
- Weight of the cut: W
- Electric phase and quadrature signals at 10 different frequencies: {Ij Qj}

The excitation frequencies used were: 25 kHz, 40 kHz, 50 kHz, 85 kHz, 100 kHz, 200 kHz, 300 kHz, 400 kHz, 600 kHz and 850 kHz.

By means of models of linear correlation it can be seen that the induction spectrum is correlated to the target variable (IMF%), and particularly the relationships between quadrature signals at different frequencies. For example, a simple linear model using only the weight and quotient $Q_{850}/Q_{40}$ provides correlations of Adj. $R^2$ = 0.76 and RMSE=0.3

$$IMF\ [\%] = 5.318 - (4.100 \cdot Q_{850}/Q_{40})/W$$

As in previous cases, the inclusion of all the variables considered in the model considerably improves the correlation. Particularly, a model based on partial least squares regression using 5 main components allows improving the adjusted coefficient of determination (Adj. $R^2$ = 0.85), reducing the prediction error to RMSE = 0.2. Figure 8 shows the results obtained based on this model.

**Claims**

1. A method for determining quality parameters in meat products comprising the steps of

    [a] providing at least one transmitter coil (2) and at least one receiver coil (4),
    [b] generating a plurality of primary time-variable electromagnetic fields at different excitation frequencies by means of said at least one transmitter coil (2),
    [c] subjecting said meat product (50) to the effects of said plurality of primary electromagnetic fields for generating currents induced in said meat product (50), and establishing a plurality of secondary electromagnetic fields,
    [d] detecting the total electromagnetic field for each of said excitation frequencies by means of said at least one receiver coil (4),
    [e] obtaining from said receiver coil (4) models of correlation comprising the in-phase and quadrature components of each of said total electromagnetic fields for each of said excitation frequencies,
    [f] obtaining an estimate of said quality parameters of said meat product (50) based on said in-phase and/or quadrature components of the total electromagnetic fields, using pre-established models of correlation,
    [g] where said models of correlation have been obtained from prior experimental tests,
    **characterized in that**
    [h] said models of correlation are models of correlation between

        [i] said in-phase and/or quadrature components of the total electromagnetic fields and
        [ii] at least one parameter of the group consisting of water-holding capacity, salt uptake in lean and/or intramuscular fat content based on each of said models of correlation.

2. The method for determining quality parameters in meat products according to claim 1, **characterized in that** it further comprises the steps of

    - obtaining the initial weight of said meat product (50),
    - adding said initial weight to said models of correlation and
    - obtaining an estimated value of the water-holding capacity and/or salt uptake in lean and/or intramuscular fat content of said meat product (50) based on said models of correlation.

3. The method for determining quality parameters in meat products according to claim 2, **characterized in that** it further comprises the steps of

    - obtaining the weight loss after salting as a difference between the initial weight of said meat product (50) and the final weight after salting,
    - adding said weight loss to said models of correlation, and
    - obtaining an estimated value of the salt uptake in lean of said meat product (50) based on said models of correlation.

4. The method for determining quality parameters in meat products according to any of claims 1 to 3, **characterized in that** it comprises the steps of

- obtaining the initial percentage by weight of lean and/or fat of said meat product (50),
- adding said initial percentage by weight of lean and/or fat to said models of correlation and
- obtaining an estimated value of the water-holding capacity and/or salt uptake in lean and/or intramuscular fat content of said meat product (50) based on said models of correlation.

5. The method for determining quality parameters in meat products according to any of claims 1 to 4, **characterized in that** it comprises the step of providing first and second transmitter coils (2) and first and second receiver coils (4), and **in that** the primary electromagnetic fields generated by said two transmitter coils (2) have a 0 or 180° phase shift, depending on the winding direction of the wires thereof, and **in that** the estimate of the secondary electromagnetic field is made based on adding or subtracting the secondary electromagnetic fields obtained from said two receiver coils (4).

6. The method for determining quality parameters in meat products according to claim 1, **characterized in that** said different excitation frequencies are comprised in a range comprised between 1 kHz to 100 MHz.

7. The method for determining quality parameters in meat products according to any of claims 1 to 6, **characterized in that** it comprises the steps of

- obtaining the total electromagnetic field in the absence of a meat product (50) by means of determining in said at least one receiver coil (4) the in-phase and quadrature components of the total electromagnetic field in the absence of said meat product (50),
- determining the phase shift between the total electromagnetic field and the primary electromagnetic field in the absence of said meat product (50), and
- correcting said phase shift.

8. The method for determining quality parameters in meat products according to claim 7, **characterized in that** it additionally comprises the steps of

- obtaining the total electromagnetic field in the absence of a meat product (50) by means of determining in said at least one receiver coil (4) the in-phase and/or quadrature component of said total electromagnetic field in the absence of said meat product (50),
- generating an electric compensation signal with a phase and a quadrature such that they cancel out the in-phase and quadrature components of the total electromagnetic field in said at least one receiver coil (4) due to the primary electromagnetic field in the absence of a meat product (50).

9. The method for determining quality parameters in meat products according to claim 8, **characterized in that** it additionally comprises the steps of:

- obtaining the amplitude of the electric compensation signal needed for cancelling out the total electromagnetic field in the absence of a meat product (50),
- normalizing the values of the in-phase and quadrature components of the total field in the presence of a meat product (50) by means of the process of dividing said in-phase and quadrature components by the value of said amplitude of the electric compensation signal,
- obtaining a model of correlation comprising said normalized in-phase and/or quadrature components of the total electromagnetic field for each of said excitation frequencies, and
- obtaining an estimated value of the water-holding capacity and/or salt uptake in lean and/or intramuscular fat content of said meat product (50) based on each of said models of correlation.

10. The method for determining quality parameters in meat products according to any of claims 1 to 9, **characterized in that** it comprises the steps of

- providing vision means (24),
- determining by means of said vision means (24) at least one morphological parameter of said meat product (50) selected from height, length, width, area and/or volume of said meat product (50), and
- adding one or several of said morphological parameters to said model of correlation, and
- obtaining an estimated value of the water-holding capacity and/or salt uptake in lean and/or intramuscular fat content of said meat product (50) based on said models of correlation.

**11.** The method for determining quality parameters in meat products according to any of claims 1 to 10, **characterized in that** it comprises the steps of

- providing vision means (24),
- determining by means of said vision means (24) at least one color parameter, expressed in the CIELAB color space, in the lean area of said meat product (50) selected from luminance (L*) and/or the red/green level (a*) and/or the blue/yellow level (b*) of said meat product (50), and
- adding one or several of said color parameters (L*a*b*) to said model of correlation, and
- obtaining an estimated value of the water-holding capacity of said meat product (50) based on said models of correlation.

**12.** The method for determining quality parameters in meat products according to claim 11, **characterized in that** it comprises a prior image processing step consisting of removing from the image obtained by said vision means (24) pixels corresponding to connective tissue and intramuscular fat before determining said color parameter.

## Patentansprüche

**1.** Verfahren zur Bestimmung von Qualitätsparametern in Fleischprodukten, dass die folgenden Schritte umfasst

[a] Bereitstellen mindestens einer Sendespule (2) und mindestens einer Empfangsspule (4),
[b] Erzeugen einer Vielzahl von primären zeitvariablen elektromagnetischen Feldern mit unterschiedlichen Anregungsfrequenzen mittels der mindestens einen Sendespule (2),
[c] Aussetzen des Fleischprodukts (50) den Wirkungen der mehreren primären elektromagnetischen Felder, um in dem Fleischprodukt (50) induzierte Ströme zu erzeugen, und Erzeugen mehrerer sekundärer elektromagnetischer Felder,
[d] Erfassen des elektromagnetischen Gesamtfeldes für jede der Anregungsfrequenzen mittels der mindestens einen Empfangsspule (4),
[e] Erhalten von Korrelationsmodellen von der Empfangsspule (4), die die In-Phaseund Quadratur-Komponenten jedes der elektromagnetischen Gesamtfelder für jede der Erregungsfrequenzen umfassen,
[f] Erhalten einer Schätzung der Qualitätsparameter des Fleischprodukts (50) auf der Grundlage der In-Phase- und/oder Quadratur-Komponenten der elektromagnetischen Gesamtfelder unter Verwendung von vorher erstellten Korrelationsmodellen,
[g] wobei die Korrelationsmodelle aus vorherigen experimentellen Tests erhalten wurden,
**dadurch gekennzeichnet, dass**
[h] die Korrelationsmodelle Korrelationsmodelle sind zwischen

[i] den In-Phase- und/oder Quadraturkomponenten der elektromagnetischen Gesamtfelder und
[ii] mindestens einem Parameter aus der Gruppe bestehend aus Wasserhaltevermögen, Salzaufnahme in Magerfleisch und/oder intramuskulärem Fettgehalt, basierend auf jedem der Korrelationsmodelle.

**2.** Verfahren zur Bestimmung von Qualitätsparametern in Fleischprodukten nach Anspruch 1, **dadurch gekennzeichnet, dass** es ferner die folgenden Schritte umfasst

- Ermitteln des Anfangsgewichts des Fleischprodukts (50),
- Addieren des Anfangsgewichts zu den Korrelationsmodellen und
- Erhalten eines Schätzwerts des Wasserhaltevermögens und/oder der Salzaufnahme in Magerfleisch und/oder des intramuskulären Fettgehalts des Fleischprodukts (50) basierend auf den Korrelationsmodellen.

**3.** Verfahren zur Bestimmung von Qualitätsparametern in Fleischprodukten nach Anspruch 2, **dadurch gekennzeichnet, dass** es ferner die folgenden Schritte umfasst

- Ermitteln des Gewichtsverlusts nach dem Salzen als Differenz zwischen dem Anfangsgewicht des Fleischprodukts (50) und dem Endgewicht nach dem Salzen,
- Addieren des Gewichtsverlustes zu den Korrelationsmodellen, und
- Erhalten eines Schätzwerts der Salzaufnahme in Magerfleisch des Fleischprodukts (50) basierend auf den Korrelationsmodellen.

4. Verfahren zur Bestimmung von Qualitätsparametern in Fleischprodukten nach einem der Ansprüche 1 bis 3, **dadurch gekennzeichnet, dass** es die folgenden Schritte umfasst

 - Ermitteln des anfänglichen Gewichtsprozentsatzes an Magerfleisch und/oder Fett des Fleischprodukts (50),
 - Addieren des anfänglichen Gewichtsprozentsatzes an Magerfleisch und/oder Fett zu den Korrelationsmodellen und
 - Erhalten eines Schätzwerts des Wasserhaltevermögens und/oder der Salzaufnahme in Magerfleisch und/oder des intramuskulären Fettgehalts des Fleischprodukts (50) basierend auf den Korrelationsmodellen.

5. Verfahren zur Bestimmung von Qualitätsparametern in Fleischprodukten nach einem der Ansprüche 1 bis 4, **dadurch gekennzeichnet, dass** es den Schritt umfasst Bereitstellen einer ersten und einer zweiten Sendespule (2) und einer ersten und einer zweiten Empfangsspule (4), und dass die von den beiden Sendespulen (2) erzeugten primären elektromagnetischen Felder eine Phasenverschiebung von 0 oder 180° aufweisen, abhängig von der Wicklungsrichtung ihrer Drähte, und dass die Schätzung des sekundären elektromagnetischen Feldes auf der Grundlage der Addition oder Subtraktion der von den beiden Empfangsspulen (4) erhaltenen sekundären elektromagnetischen Felder erfolgt.

6. Verfahren zur Bestimmung von Qualitätsparametern in Fleischprodukten nach Anspruch 1, **dadurch gekennzeichnet, dass** die verschiedenen Anregungsfrequenzen in einem Bereich zwischen 1 kHz und 100 MHz liegen.

7. Verfahren zur Bestimmung von Qualitätsparametern in Fleischprodukten nach einem der Ansprüche 1 bis 6, **dadurch gekennzeichnet, dass** es die Schritte umfasst

 - Erhalten des elektromagnetischen Gesamtfeldes in Abwesenheit eines Fleischprodukts (50) durch Bestimmen der Inphasen- und Quadraturkomponenten des elektromagnetischen Gesamtfeldes in Abwesenheit des Fleischprodukts (50) in der mindestens einen Empfangsspule (4),
 - Bestimmen der Phasenverschiebung zwischen dem elektromagnetischen Gesamtfeld und dem primären elektromagnetischen Feld in Abwesenheit des Fleischprodukts (50), und
 - Korrigieren der Phasenverschiebung.

8. Verfahren zur Bestimmung von Qualitätsparametern in Fleischprodukten nach Anspruch 7, **dadurch gekennzeichnet, dass** es zusätzlich die folgenden Schritte umfasst

 - Ermitteln des elektromagnetischen Gesamtfeldes in Abwesenheit eines Fleischproduktes (50) durch Bestimmen der In-Phase- und/oder Quadraturkomponente des elektromagnetischen Gesamtfeldes in Abwesenheit des Fleischproduktes (50) in der mindestens einen Empfangsspule (4),
 - Erzeugen eines elektrischen Kompensationssignals mit einer Phase und einer Quadratur, so dass sie die In-Phase- und Quadratur-Komponenten des elektromagnetischen Gesamtfeldes in der mindestens einen Empfangsspule (4) aufgrund des primären elektromagnetischen Feldes in Abwesenheit eines Fleischproduktes (50) auslöschen können.

9. Verfahren zur Bestimmung von Qualitätsparametern in Fleischprodukten nach Anspruch 8, **dadurch gekennzeichnet, dass** es zusätzlich die folgenden Schritte umfasst:

 - Gewinnen der Amplitude des elektrischen Kompensationssignals, das zur Auslöschung des elektromagnetischen Gesamtfeldes in Abwesenheit eines Fleischproduktes (50) benötigt wird,
 - Normalisieren der Werte der In-Phase- und Quadratur-Komponenten des Gesamtfeldes bei Vorhandensein eines Fleischproduktes (50) mittels des Verfahrens des Teilens der In-Phase- und Quadratur-Komponenten durch den Wert der Amplitude des elektrischen Kompensationssignals,
 - Erhalten eines Korrelationsmodells, das die normalisierten In-Phase- und/oder Quadratur-Komponenten des elektromagnetischen Gesamtfeldes für jede der Anregungsfrequenzen umfasst, und
 - Erhalten eines Schätzwerts der Wasserhaltekapazität und/oder der Salzaufnahme im Magerfleisch und/oder des intramuskulären Fettgehalts des Fleischprodukts (50) auf der Grundlage jedes der Korrelationsmodelle.

10. Verfahren zur Bestimmung von Qualitätsparametern in Fleischprodukten nach einem der Ansprüche 1 bis 9, **dadurch gekennzeichnet, dass** es die folgenden Schritte umfasst

 - Bereitstellen von Sichtmitteln (24),

- Bestimmen mindestens eines morphologischen Parameters des Fleischprodukts (50), ausgewählt aus Höhe, Länge, Breite, Fläche und/oder Volumen des Fleischprodukts (50), mit Hilfe der Sichtmittel (24), und
- Hinzufügen eines oder mehrerer der morphologischen Parameter zu dem Korrelationsmodell, und
- Erhalten eines Schätzwerts des Wasserhaltevermögens und/oder der Salzaufnahme im Magerfleisch und/oder intramuskulären Fettgehalts des Fleischprodukts (50) auf der Grundlage der Korrelationsmodelle.

11. Verfahren zur Bestimmung von Qualitätsparametern in Fleischprodukten nach einem der Ansprüche 1 bis 10, **dadurch gekennzeichnet, dass** es die folgenden Schritte umfasst

- Bereitstellen von Sichtmitteln (24),
- Bestimmen mittels der Sichtmittel (24) mindestens eines Farbparameters, ausgedrückt im CIELAB-Farbraum, im mageren Bereich des Fleischprodukts (50), ausgewählt aus der Luminanz (L*) und/oder dem Rot/Grün-Pegel (a*) und/oder dem Blau/Gelb-Pegel (b*) des Fleischprodukts (50), und
- Hinzufügen eines oder mehrerer der Farbparameter (L*a*b*) zu dem Korrelationsmodell, und
- Erhalten eines Schätzwerts der Wasserhaltekapazität des Fleischprodukts (50) auf der Grundlage der Korrelationsmodelle.

12. Verfahren zur Bestimmung von Qualitätsparametern in Fleischprodukten nach Anspruch 11, **dadurch gekennzeichnet, dass** es einen vorherigen Bildverarbeitungsschritt umfasst, der darin besteht, dass aus dem durch die Bildverarbeitungseinrichtung (24) erhaltenen Bild vor der Bestimmung des Farbparameters Pixel entfernt werden, die Bindegewebe und intramuskulärem Fett entsprechen.

## Revendications

1. Procédé de détermination de paramètres de qualité dans des produits carnés comprenant les étapes consistant à

[a] prévoir au moins une bobine émettrice (2) et au moins une bobine réceptrice (4),
[b] générer une pluralité de champs électromagnétiques primaires variables dans le temps à différentes fréquences d'excitation au moyen de ladite au moins une bobine émettrice (2),
[c] soumettre ledit produit carné (50) aux effets de ladite pluralité de champs électromagnétiques primaires pour générer des courants induits dans ledit produit carné (50), et établir une pluralité de champs électromagnétiques secondaires,
[d] détecter le champ électromagnétique total pour chacune desdites fréquences d'excitation au moyen de ladite au moins une bobine réceptrice (4),
[e] obtenir à partir de ladite bobine réceptrice (4) des modèles de corrélation comprenant les composantes en phase et en quadrature de chacun desdits champs électromagnétiques totaux pour chacune desdites fréquences d'excitation,
[f] obtenir une estimation desdits paramètres de qualité dudit produit carné (50) sur la base desdites composantes en phase et/ou en quadrature des champs électromagnétiques totaux, à l'aide de modèles de corrélation préétablis,
[g] où lesdits modèles de corrélation ont été obtenus à partir d'essais expérimentaux antérieurs,
**caractérisé en ce que**
[h] lesdits modèles de corrélation sont des modèles de corrélation entre

[i] lesdites composantes en phase et/ou en quadrature des champs électromagnétiques totaux, et
[ii] au moins un paramètre du groupe consistant en la capacité de rétention d'eau, l'absorption de sel dans la viande maigre et/ou la teneur en gras intramusculaire sur la base de chacun desdits modèles de corrélation.

2. Procédé de détermination de paramètres de qualité dans des produits carnés selon la revendication 1, **caractérisé en ce qu'il** comprend en outre les étapes consistant à

- obtenir le poids initial dudit produit carné (50),
- ajouter ledit poids initial auxdits modèles de corrélation, et
- obtenir une valeur estimée de la capacité de rétention d'eau et/ou d'absorption de sel dans la viande maigre et/ou la teneur en gras intramusculaire dudit produit carné (50) sur la base desdits modèles de corrélation.

3. Procédé de détermination de paramètres de qualité dans des produits carnés selon la revendication 2, **caractérisé en ce qu'il** comprend en outre les étapes consistant à

- obtenir la perte de poids après salage en tant que différence entre le poids initial dudit produit carné (50) et le poids final après salage,
- ajouter ladite perte de poids auxdits modèles de corrélation, et
- obtenir une valeur estimée de l'absorption de sel dans la viande maigre dudit produit carné (50) sur la base desdits modèles de corrélation.

4. Procédé de détermination de paramètres de qualité dans des produits carnés selon l'une des revendications 1 à 3, **caractérisé en ce qu'il** comprend les étapes consistant à

- obtenir le pourcentage initial en poids de viande maigre et/ou de matière grasse dudit produit carné (50),
- ajouter ledit pourcentage initial en poids de viande maigre et/ou de matière grasse auxdits modèles de corrélation, et
- obtenir une valeur estimée de la capacité de rétention d'eau et/ou d'absorption de sel dans la viande maigre et/ou la teneur en gras intramusculaire dudit produit carné (50) sur la base desdits modèles de corrélation.

5. Procédé de détermination de paramètres de qualité dans des produits carnés selon l'une des revendications 1 à 4, **caractérisé en ce qu'il** comprend l'étape consistant à prévoir des première et deuxième bobines émettrices (2) et des première et deuxième bobines réceptrices (4), et **en ce que** les champs électromagnétiques primaires générés par lesdites deux bobines émettrices (2) ont un déphasage de 0 ou 180°, en fonction de la direction d'enroulement de leurs fils, et **en ce que** l'estimation du champ électromagnétique secondaire est faite sur la base de l'addition ou de la soustraction des champs électromagnétiques secondaires obtenus à partir desdites deux bobines réceptrices (4).

6. Procédé de détermination de paramètres de qualité dans des produits carnés selon la revendication 1, **caractérisé en ce que** lesdites différentes fréquences d'excitation sont comprises dans une plage allant de 1 kHz à 100 MHz.

7. Procédé de détermination de paramètres de qualité dans des produits carnés selon l'une des revendications 1 à 6, **caractérisé en ce qu'il** comprend les étapes consistant à

- obtenir le champ électromagnétique total en l'absence d'un produit carné (50) en déterminant dans ladite au moins une bobine réceptrice (4) les composantes en phase et en quadrature du champ électromagnétique total en l'absence dudit produit carné (50),
- déterminer le déphasage entre le champ électromagnétique total et le champ électromagnétique primaire en l'absence dudit produit carné (50), et
- corriger ledit déphasage.

8. Procédé de détermination de paramètres de qualité dans des produits carnés selon la revendication 7, **caractérisé en ce qu'il** comprend en outre les étapes consistant à

- obtenir le champ électromagnétique total en l'absence de produit carné (50) en déterminant dans ladite au moins une bobine réceptrice (4) la composante en phase et/ou en quadrature dudit champ électromagnétique total en l'absence dudit produit carné (50),
- générer un signal de compensation électrique avec une phase et une quadrature telles qu'elles annulent les composantes en phase et en quadrature du champ électromagnétique total dans ladite au moins une bobine réceptrice (4) due au champ électromagnétique primaire en l'absence d'un produit carné (50).

9. Procédé de détermination de paramètres de qualité dans des produits carnés selon la revendication 8, **caractérisé en ce qu'il** comprend en outre les étapes consistant à :

- obtenir l'amplitude du signal de compensation électrique nécessaire pour annuler le champ électromagnétique total en l'absence d'un produit carné (50),
- normaliser les valeurs des composantes en phase et en quadrature du champ total en présence d'un produit carné (50) au moyen du processus de division desdites composantes en phase et en quadrature par la valeur de ladite amplitude du signal de compensation électrique,
- obtenir un modèle de corrélation comprenant lesdites composantes en phase et/ou en quadrature normalisées du champ électromagnétique total pour chacune desdites fréquences d'excitation, et

- obtenir une valeur estimée de la capacité de rétention d'eau et/ou d'absorption de sel dans la viande maigre et/ou la teneur en gras intramusculaire dudit produit carné (50) sur la base de chacun desdits modèles de corrélation.

10. Procédé de détermination de paramètres de qualité dans des produits carnés selon l'une des revendications 1 à 9, **caractérisé en ce qu'il** comprend les étapes consistant à

- prévoir un moyen de visualisation (24),
- déterminer au moyen dudit moyen de visualisation (24) au moins un paramètre morphologique dudit produit carné (50) choisi parmi la hauteur, la longueur, la largeur, la surface et/ou le volume dudit produit carné (50), et
- ajouter un ou plusieurs desdits paramètres morphologiques audit modèle de corrélation, et
- obtenir une valeur estimée de la capacité de rétention d'eau et/ou d'absorption de sel dans la viande maigre et/ou la teneur en gras intramusculaire dudit produit carné (50) sur la base desdits modèles de corrélation.

11. Procédé de détermination de paramètres de qualité dans des produits carnés selon l'une des revendications 1 à 10, **caractérisé en ce qu'il** comprend les étapes consistant à

- prévoir un moyen de visualisation (24),
- déterminer au moyen dudit moyen de visualisation (24) au moins un paramètre de couleur, exprimé dans l'espace colorimétrique CIELAB, dans la zone maigre dudit produit carné (50) choisi parmi la luminance (L*) et/ou le niveau rouge/vert (a*) et/ou le niveau bleu/jaune (b*) dudit produit carné (50), et
- ajouter un ou plusieurs desdits paramètres de couleur (L*a*b*) audit modèle de corrélation, et
- obtenir une valeur estimée de la capacité de rétention d'eau dudit produit carné (50) sur la base desdits modèles de corrélation.

12. Procédé de détermination de paramètres de qualité dans des produits carnés selon la revendication 11, **caractérisé en ce qu'il** comprend une étape préalable de traitement d'image consistant à retirer de l'image obtenue par lesdits moyens de visualisation (24) des pixels correspondant au tissu conjonctif et au gras intramusculaire avant de déterminer ledit paramètre de couleur.

EP 3 098 597 B1

FIG.1

FIG.2

16

FIG.3

FIG.4

FIG.5

FIG.6

FIG.7

FIG.8

FIG.9

FIG.10

**EP 3 098 597 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0375366 A2